# EUROPEAN PATENT SPECIFICATION

(11) **EP 2 574 926 B1**
(45) Date of publication and mention of the grant of the patent: **02.09.2015**
(21) Application number: 12184646.3
(22) Date of filing: 17.09.2012
(51) Int. Cl.: G01N 33/543, G01N 33/553

(54) **Chromatographic kit and chromatography method**
Chromatografiekit und Chromatografieverfahren
Procédé de chromatographie et kit chromatographique

(30) Priority: 29.09.2011 JP 2011214342; 07.03.2012 JP 2012050232; 30.08.2012 JP 2012189799
(43) Date of publication of application: 03.04.2013
(73) Proprietor: FUJIFILM Corporation, Tokyo 106-8620 (JP)
(72) Inventor: Wada, Atsuhiko, Kanagawa, 258-8577 (JP); Mori, Mikinaga, Kanagawa, 258-8577 (JP)
(74) Representative: Hoffmann Eitle

(56) References cited:
- EP-A1- 1 054 259
- EP-A1- 2 330 421
- US-A1- 2009 181 470
- FREDERICK C B ET AL: "A chromatographic technique for the analysis of oxidized metabolites: Application to carcinogenic N-hydroxyarylamines in urine", ANALYTICAL BIOCHEMISTRY, ACADEMIC PRESS INC, NEW YORK, vol. 118, no. 1, 15 November 1981 (1981-11-15), pages 120-125, XP024817132, ISSN: 0003-2697, DOI: 10.1016/0003-2697(81)90166-4 [retrieved on 1981-11-15]

## Description

### BACKGROUND OF THE INVENTION

### 1. Field of the Invention

The present invention relates to a chromatographic kit and a chromatography method in which a signal amplification operation is performed in order to increase detection sensitivity.

### 2. Description of the Related Art

An immunochromatography method is generally often used among immunoassay methods since the operation is simple and a sample can be measured in a short time. As an immune reaction used in an immunochromatography method, a competition-type reaction or a sandwich-type reaction is widely used. Of these, in an immunochromatography method, the sandwich-type reaction is standard and, in a typical example, the following operation is carried out in order to detect the test substance consisting of an antigen in a sample. First, a chromatographic carrier having a reaction site is prepared by immobilizing fine particles sensitized by an antibody of an antigen which is the test substance on the chromatographic carrier as the solid-phase fine particles, or by directly immobilizing this antibody itself on the chromatographic carrier. Meanwhile, sensitized labeling fine particles are prepared by sensitizing the antibody which can specifically bind to the test substance to labeling fine particles. These sensitized labeling fine particles, together with the sample, move chromatographically on the chromatographic carrier. From the above operation, the immobilized antibody becomes the immobilizing reagent in the reaction site formed in the chromatographic carrier and the sensitized labeling fine particles specifically bind to the immobilizing reagent through the antigen which is the test substance. As a result, the presence, absence or the amount of the test substance in the sample may be measured by visually determining the presence, absence, or the degree of the signal generated by the sensitized labeling fine particles being captured in the reaction site.

In an immunochromatography method, there is a case in which a method of amplifying a detection signal is used in order to avoid a problem in that an antigen (false-negative) is not detected due to low sensitivity. As the method of signal amplification, an enzyme such as alkaline phosphatase or peroxidase may be used as a label. However, a method of carrying out detection by sensitization using a compound including silver and a reducing agent for silver ions on a label selected from the group consisting of colloidal metal labels and metal sulfide labels (silver amplification) may also be used. Immunochromatography using the amplification described above is disclosed in JP2002-202307A, JP2010-71827A, JP2011-99724A and Journal of Chromatography, 878 (2010) 271 to 277, and particularly, carrying out amplification using an amplification liquid made of two liquids is disclosed in JP2010-71827A and JP2011-99724A. However, in JP2010-71827A and JP2011-99724A, means for determining the beginning timing of the second liquid addition when the amplification is carried out using an amplification liquid made of two liquids is not disclosed.

US-A1-2009/0181470 provides a measurement kit which comprises a first developing member for supplying a first developing solution and a second developing member for supplying a second developing solution, wherein the developing direction of the first developing solution is allowed to intersect with the developing direction of the second developing solution, so that development is carried out by developing the first and second developing solutions in different developing directions, and a water absorbent portion is established on the downstream of the developing directions.

The first developing solution is a solution containing an analyte and the second developing solution is a solution containing an amplification solution. The kits may further comprise a label substance, such as a metal colloid, modified with a second substance that binds to the analyte or with a compound having a portion similar to the analyte.

### SUMMARY OF THE INVENTION

As described above, in a chromatography method, amplification is sometimes carried out using two liquids which are a liquid catalyzing the amplification and a liquid carrying out the amplification. In this case, an area which needs to be amplified on a chromatographic carrier must be filled with a first liquid, however, the flow rate of the first liquid (a reducing agent, and the like) is changed due to the length of a support or a flow rate and the temperature of an external environment, and the like. Therefore, there has been a problem such that, if the addition timing of a second liquid is specified as a time, amplification failure occurs since the second liquid is added before being filled with the first liquid, or, in order to prevent amplification failure, it is necessary to set the addition timing of the second liquid later than necessary. In addition, situations are also postulated such that a required number of clocks cannot be prepared when measurements are carried out outdoors or a large number of analytes are measured in a short period of time.

That is, as solution to problems, the present invention has been made to provide a chromatographic kit and a chromatography method in which an occurrence of amplification failure due to addition of a second liquid before being filled with a first liquid can be prevented since the site which needs to be amplified on a chromatographic carrier being filled with the first liquid is easily confirmed.

The inventors, as a result of intensive studies to solve the problem described above, have succeeded in preventing the occurrence of amplification failure by providing means by which the addition timing of a liquid carrying out the amplification is visually informed, in a chromatographic kit and a chromatography method in which amplification is carried out using two liquids which are a liquid catalyzing the amplification and a liquid carrying out the amplification. The present invention has been completed based on these findings.

According to a first aspect, the present invention provides a chromatographic kit for detecting a test substance in a sample, the kit comprising:
(1) a labelling substance holding area, which is an area having a labelling substance bound to a first binding substance, wherein the labelling substance is a colloidal metal and the first binding substance is a substance capable of binding to the test substance;
(2) a labelling substance capturing area, which is an area having a second binding substance, wherein the second binding substance is a substance capable of binding to the test substance or to the first binding substance;
(3) first and second amplification reagents, which are capable of amplifying a signal of the labelling substance in order to aid the detection of the labelling substance; and
(4) an area having a colour developing reagent, which is a reagent capable of detecting the first amplification reagent;
wherein the first amplification reagent is a reducing agent for silver ions that includes divalent iron ions, the second amplification reagent is a compound including silver, and the colour developing agent is a compound having a phenanthroline skeleton, a compound having a bipyridine skeleton or a salton-based colour developing reagent.

Preferably the colour developing reagent is a compound having a phenanthroline skeleton.

Preferably the colloidal metal is colloidal gold.

In the chromatographic kit of the above first aspect, it is preferred that:
the labelling substance holding area is a pad holding the labelling substance;
the labelling substance capturing area is located in a membrane in which the second binding substance is immobilized;
the colour developing reagent is carried on the membrane in which the second binding substance is immobilized; and
the kit further comprises a water absorption pad.

In this case, it is preferred that the colour developing reagent is a colour developing reagent which does not substantially move in the binding substance immobilized membrane when any of an aqueous solution including the test sample or an aqueous solution including the first amplification reagent is developed.

According to a second aspect, the present invention provides a chromatography method using the chromatographic kit according to the above first aspect, the method comprising:
(1) forming a complex between the test substance and the labelling substance bound to the first binding substance, and then capturing the labelling substance in the labelling substance capturing area;
(2) filling the labelling substance capturing area with the first amplification reagent from an upstream side of the labelling substance capturing area to a downstream side of the labelling substance capturing area;
(3) confirming that the labelling substance capturing area is filled with the first amplification reagent by detecting physical or chemical changes in the area having the colour developing reagent; and
(4) filling the labelling substance capturing area with the second amplification reagent after confirming that the labelling substance capturing area is filled with the first amplification reagent;
wherein the first amplification reagent is a reducing agent for silver ions that includes divalent iron ions, the second amplification reagent is a compound including silver, and the colour developing agent is a compound having a phenanthroline skeleton or a compound having a bipyridine skeleton or a salton-based colour developing reagent.

According to the chromatographic kit and the chromatography method of the present invention, an occurrence of amplification failure can be prevented by providing means by which the addition timing of a liquid carrying out the amplification is visually informed.

### BRIEF DESCRIPTION OF THE DRAWINGS

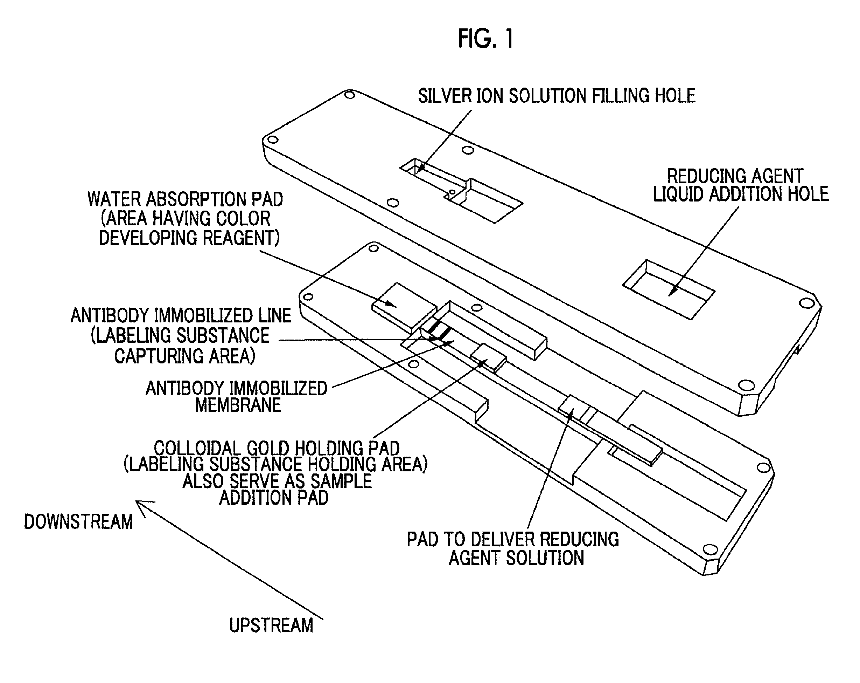

Fig. 1 shows an embodiment of a kit used in Example 1. In an upper member constituting the outside of the kit of the present invention, a silver ion solution filling hole which is a second amplification reagent is provided. In a lower member, a pad in order to deliver the liquid of a reducing agent solution which is a first amplification reagent, a colloidal gold holding pad which is a labeling substance holding area, an antibody immobilized membrane having an antibody immobilized line which is a labeling substance capturing area, and a water absorption pad which is an area having a color developing reagent are disposed in this order on an adhesive sheet. As shown in Fig. 1, upstream and downstream are defined with respect to the direction of development of the test sample including the test substance.

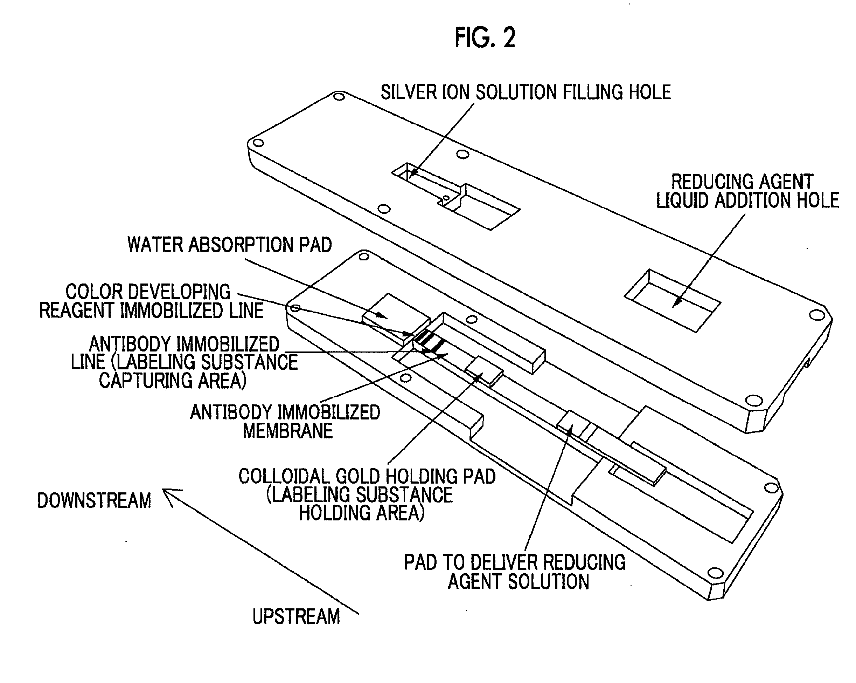

Fig. 2 shows embodiments of kits used in Example 2 and Example 3. In an upper member constituting the outside of the kit of the present invention, a silver ion solution filling hole which is a second amplification reagent and a reducing agent solution addition hole which is a first amplification reagent are provided. In a lower member, a pad in order to deliver the liquid of a reducing agent solution which is a first amplification reagent, a colloidal gold holding pad which is a labeling substance holding area, an antibody immobilized membrane having an antibody immobilized line which is a labeling substance capturing area and a color developing reagent immobilized line which is an area having a color developing agent, and a water absorption pad are disposed in a T on an adhesive sheet. As with Fig. 1, upstream and downstream are defined with respect to the direction of development of the test sample including the test substance.

### DESCRIPTION OF THE PREFERRED EMBODIMENTS

In the chromatographic kit and the chromatography method of the present invention, visually determining the addition timing of two types of the amplification reagents used in carrying out the amplification with the aim of increasing the detection sensitivity of the test substance is possible.

### 1. Color Developing Reagent for Detecting First Amplification Reagent

The chromatographic kit of the present invention has an area having a color developing reagent for detecting a first amplification reagent of two types of the amplification reagents used to amplify the signal of a labeling substance.

In the present invention, as a color developing reagent for detecting the first amplification reagent, a compound is used which develops color by reacting with ions. The first amplification reagent which is a reducing agent for silver ions that includes divalent iron ions (Fe²⁺) will be described later in this specification. A compound which develops color by reacting with Fe²⁺ ions is used as the color developing reagent. The colour developing agent which develops color by reacting with Fe²⁺ ions may form a complex with Fe²⁺ ions. This agent is a compound having a phenanthroline skeleton [for example, 1,10-phenanthroline, 5-methylphenanthroline, 5-nitrophenanthroline, bathophenanthroline (4,7-diphenyl-1,10-phenanthroline) or bathophenanthroline disulfonic acid] or a compound having a bipyridine skeleton [for example, 2,2'-bipyridine]. Preferably the compound has a phenanthroline skeleton.
Alternatively, a salton-based colour developing reagent which changes color shades due to a structural change caused by protons in order to detect the first amplification reagent may be used if the pH of an aqueous solution including a test sample and an aqueous solution including the first amplification reagent are different. In particular, if the aqueous solution including the first amplification reagent is acidic (smaller than pH 7 and the concentration of protons is high), a compound which develops color by reacting with H⁺ ions which is a color developing reagent well known as a pH indicator for the acidic region (a salton-based color developing reagent such as thymol blue, bromocresol green, bromocresol purple and bromothymol blue) may be used in accordance with the pH of an aqueous solution including the amplification reagent. Among these, 1,10-phenanthroline, bathophenanthroline or bromocresol green is more preferably used.

Since it is preferable that the color reagent not move on a binding substance immobilized membrane when an aqueous solution including the test sample or an aqueous solution including the first amplification reagent is developed, LogP (a partition coefficient in water and octanol) of the color developing reagent is preferably 4.0 or more, and more preferably 5.0 or more. As the LogP, an actual measured value may be used, however, a calculated value obtained from a chemical structure, or the like, may also be used as a simple method of determination. The method of calculating the LogP is preferably a calculation method used in ChemDrawPro version 12 by PerkinElmer, Inc. (formerly CambridgeSoft Corporation). Responsiveness of a typical color developing reagent and LogP (according to ChemDrawPro version 12) is shown in Table 1 below.

**[Table 1]**

| Compound Name | Responsiveness | LogP |
|---|---|---|
| 2,2'-Bipyridine | Fe²⁺ response | 1.88 |
| Bathophenanthroline disulfonic acid | Fe²⁺ response | 0.52 |
| 1,10-Phenanthroline | Fe²⁺ response | 2.2 |
| 5-Methylphenanthroline | Fe²⁺ response | 2.69 |
| 5-Nitrophenanthroline | Fe²⁺ response | 2.34 |
| Thymol Blue | pH response | 4.01 |
| Bathophenanthroline | Fe²⁺ response | 5.55 |
| Bromocresol Green | pH response | 7.99 |
| Bromocresol Purple | pH response | 6.33 |
| Bromothymol Blue | pH response | 8.8 |

Methods of holding the color developing reagent in the chromatographic kit include a method in which a water absorption pad which will be described later is dipped in a color developing reagent solution and dried under reduced pressure, a method in which a color developing agent is coated in a line shape further toward a downstream direction than a labeling substance replenishing area of a binding substance immobilized membrane, or the like.

If the color developing reagent substantially moves in the binding substance immobilized membrane when any of the aqueous solution including the test sample or the aqueous solution including the first amplification reagent is developed, it is preferable that the color developing reagent be included in the water absorption pad and used.

If the color developing reagent substantially does not move in the binding substance immobilized membrane when any of the aqueous solution including the test sample or the aqueous solution including the first amplification reagent is developed, it is preferable that the color developing reagent be carried on the binding substance immobilized membrane having a labeling substance capturing area.

An embodiment in which the color developing reagent is carried on the binding substance immobilized membrane is more preferable in the present invention in order to make it possible to display the reaching of the first amplification reagent to the labeling substance replenishing area with a smaller time lag.

In the present invention, having the labeling substance holding area as an upstream direction and the labeling substance replenishing area as a downstream direction are defined as an upstream direction and a downstream direction with respect to the development direction of the test sample including the test substance when the test sample is developed using capillary action or a water absorption force in a case of using a water absorption pad, and the like. In the specific embodiment of the present invention, as shown in Fig. 1 and Fig. 2, the labeling substance holding area is defined as an upstream direction and the labeling substance replenishing area as a downstream direction when the test sample and the like are developed from the labeling substance holding area toward the labeling substance replenishing area.

In the present invention, the labeling substance capturing area being filled with the first amplification reagent is confirmed by developing the first amplification reagents of the two amplification reagents used to amplify the signal of the labeling substance captured in the labeling substance capturing area from the upstream direction of the labeling substance capturing area to the downstream direction of the labeling substance capturing area and detecting the physical or chemical changes in the area having a color developing reagent. As the physical or chemical changes in the area having a color reagent, color developments, fluorescence changes or the like, caused by the reaction between the first amplification reagent and the color developing reagent may be detected. It is preferable that the color development be detected. Such physical or chemical changes may be detected visually or detected using detection devices.

### 2. Chromatograph

In general, a chromatography method is a method which simply, quickly, and specifically determines and measures a test substance in the following manner. That is, a binding substance immobilized membrane capable of having at least one labeling substance capturing area including an immobilized reagent capable of binding to the test substance (corresponding to a second binding substance of test substance or a binding substance of the following first binding substance; specifically an antibody, an antigen, and the like) is used as a stationary phase. On this binding substance immobilized membrane, a liquid including the labeling substance modified with the first binding substance of the test substance is used as a mobile phase, and along with the mobile phase moving through the binding substance immobilized membrane chromatographically, the test substance and the labeling substance specifically bind and reach the labeling substance capturing area. In the labeling substance capturing area, it is a method which qualitatively and quantitatively analyzes the presence of the test substance in the test sample visually or using suitable devices, using the fact that the labeling substance is concentrated on the second binding substance or the binding substance of the first binding substance only when the test substance is present in the test sample by the complex of the test substance and the labeling substance specifically binding to the immobilized second binding substance or the binding substance of the first binding substance.

A kit which carries out the chromatography method in the present invention uses two types of amplification reagents to amplify the signal of the labeling substance inside, a compound containing silver and a reducing agent for silver ions, and the signal is amplified according to an amplification reaction using the complex of the test substance which binds to the immobilizing reagent on the labeling substance capturing area and the labeling substance being made to be a core, and as a result, high-sensitivity may be achieved. According to the present invention, quick and highly sensitive chromatography may be performed.

### 3. Test Sample

The test sample which can be analyzed using the chromatographic kit of the present invention is not particularly limited as long as the sample has a possibility of including the test substance and, for example, a biological sample, particularly body fluid of an animal (particularly a human) (for example, blood, serum, plasma, cerebrospinal fluid, lacrimal fluid, sweat, urine, pus, nasal discharge, or sputum) or an excretion (for example, excrement), organ, tissue, mucous membrane or skin, abraded analyte considered to contain these (swab), gargle liquid, or animals and plants themselves or dried bodies thereof may be included. The test substance may include physiologically active substances such as natural substances, toxins, hormones or pesticides, environmental pollutants, virus, antigens, antibodies, or the like.

### 4. Pretreatment of Test Sample

In the measurement method using the chromatographic kit of the present invention, the test sample may be used as is, or as a form of extracted solution obtained from extracting the test sample using a suitable extracting solvent, furthermore, as a form of diluted solution obtained from diluting the extracted solution using a suitable diluting agent, or as a concentrated form of the extracted solution using a suitable method. As the extracting solvent used in the present invention, a solvent used in common immunological analysis (for example, water, physiological sodium chloride solution, buffer solution or the like) or a water-miscible organic solution in which the antigen-antibody reaction may be directly carried out by being diluted with such solvents may be used.

### 5. Configuration

In the chromatographic kit of the present invention, a chromatographic strip may be installed and used. The chromatographic strip which may be used is not particularly limited as long as it is a chromatographic strip which can be used in common chromatography methods.

The chromatographic strip which may be used in the present invention has a labeling substance holding area and a labeling substance replenishing area from an upstream direction toward a downstream direction of the development of a test sample including a test substance, and also has an area having a color developing reagent. As more preferable embodiments of the present invention, an embodiment in which an area having a color developing reagent is located in a downstream direction of the labeling substance replenishing area is used, and furthermore, an embodiment in which a sample addition pad, a labeling substance holding pad having a labeling substance holding area (for example, a colloidal gold antibody holding pad), a binding substance immobilized membrane (for example, an antibody immobilized membrane having a labeling substance capturing area) and a water absorption pad are disposed in this order on an adhesive sheet is preferably used. The binding substance immobilized membrane includes a labeling substance capturing area which is an area in which an antibody or an antigen specifically bound to a test substance is immobilized, and may further include a control zone (also may be described as a control area), which is an area in which an antibody or an antigen for control is immobilized if desired.

The labeling substance holding pad having a labeling substance holding area which may be used in the present invention may be prepared by preparing a suspension containing the labeling substance, coating the suspension on a suitable water absorption pad (for example, a glass fiber pad), and then drying it.

### 5-1. Labeling Substance

Colloidal metal is used as the labeling substance in the present invention. All well-known coloring colloidal metals in the related art may be used as coloring particles for labeling, and for example, colloidal gold, colloidal silver, colloidal platinum, colloidal iron, colloidal aluminum hydroxide, colloidal composites of these, and the like, may be included, and particularly, in suitable particle diameters, colloidal gold showing a red color and colloidal silver showing a yellow color is preferable, and among these, colloidal gold is most preferable. When colloidal gold particles are used as a colloidal metal, commercially available products may be used. Alternatively, colloidal gold particles may be prepared in the usual manner, for example, a method in which chloroauric acid is reduced with sodium citrate (Nature Physical Science, 241 (1973) 20, and the like).

The average particle diameter of the colloidal metal is preferably approximately 1 to 500 nm, more preferably 1 to 50 nm, and particularly preferably 1 to 15 nm. The average particle diameter of the colloidal metal used in the present invention may be measured using a commercially available particle size distribution analyzer, and the like. As methods of measuring particle size distribution, an optical microscopy method, a confocal laser microscopy method, an electron microscopy method, an atomic force microscopy method, a static light scattering method, a laser diffraction method, a dynamic light scattering method, a centrifugal sedimentation method, an electrical pulse measurement method, a chromatography method, an ultrasonic attenuation method, and the like, are known, and devices that support each principle are commercially available.

A dynamic light scattering method is preferably used in the present invention considering the particle range and readiness of measurement. As the commercially available measuring devices using dynamic light scattering, a nano track UPA (Nikkiso Co., Ltd.), a dynamic light scattering-type particle size distribution measuring device LB-550 (Horiba, Ltd.), a dense system particle size analyzer FPAR-1000 (Otsuka Electronics Co., Ltd.) and the like, may be included, and in the present invention, the average particle diameter is determined as the value of a median diameter (d=50) measured at a measurement temperature of 25°C.

According to the present invention, a signal of the metal-based label may be amplified in chromatography using a colloidal gold label or a metal sulfide label, other metal alloy labels (hereinafter, sometimes referred to as a metal-based label), and a polymer particle label including a metal as the labeling substance for detection. Specifically, if the silver ions supplied from a compound including silver such as an inorganic silver salt or an organic silver salt is brought into contact with a reducing agent for silver ions and silver particles are produced by the reduction of the silver ions using the reducing agent after the complex of the test substance and the labeling substance for detection is formed, a highly sensitive analysis of the test substance may be performed by the metal-based label being amplified since the silver particles are deposited on the metal-based label with the metal-based label being a core. Therefore, in the chromatography method of the present invention, well-known chromatography methods in the related art may be used as is except that a reaction which uses silver particles produced from the reduction of silver ions using the reducing agent to deposit on the label of the immune complex is performed and the signal amplified in this way is analyzed.

### 5-2. Binding Substance

In the present invention, the labeling substance is modified with the first binding substance of the test substance. As the first binding substance, for example, any of an antibody of the test substance (antigen), an antigen of the test substance (antibody), an aptamer of the test substance (protein, low-molecular compound or the like), and a compound having an affinity for the test substance may be used.

The chromatographic kit of the present invention has (a) the second binding substance of the test substance or (b) the binding substance of the first binding substance in the labeling substance capturing area. As the second binding substance of the test substance, for example, any of an antibody of the test substance (antigen), an antigen of the test substance (antibody), an aptamer of the test substance (protein, low-molecular compound or the like), and a compound having an affinity for the test substance may be used. Also the second binding substance may be different from or the same as the first binding substance. The binding substance of the first binding substance may be a test substance itself, a compound having a site in which the first binding substance recognizes and, for example, a compound in which a derivative of the test substance and protein (for example, BSA or the like) are bound may be included.

It is preferable that the first binding substance be an antibody and/or the second binding substance be an antibody. It is more preferable that the first binding substance be an antibody, and the second binding substance be an antibody which binds to the first binding substance.

In the chromatography method of the present invention, the antibody which has specificity with respect to the test substance is not particularly limited, however, for example, an antiserum prepared from a serum of an animal immunized by the test substance, an immune globulin fraction purified from the antiserum, a monoclonal antibody obtained by cell fusion which uses spleen cells of an animal immunized by the test substance or fragments thereof [for example, F (ab')2, Fab, Fab', or Fv] may be used. The preparation of these antibodies may be performed in the usual manner.

In the present invention, for example, as the method of modifying the labeling substance using the first binding substance, for example, when a colloidal metal and a specific binding substance are bound, a method which follows well-known methods in the related art (for example The Journal of Histochemistry and Cytochemistry, 30, 7 (1982) 691 to 696) described below may be used. As specific examples, a colloidal metal and a specific binding substance (for example an antibody) are mixed for 5 minutes or more at room temperature in an appropriate buffer liquid. After the reaction, the precipitate obtained by centrifugation is dispersed into a solution including a dispersing agent such as polyethylene glycol, and a target colloidal metal labeled specific binding substance may be obtained.

### 5-3. Binding Substance Immobilized Membrane

As the binding substance immobilized membrane which may be used in the present invention, a porous carrier is preferable. In particular, a nitrocellulose membrane, a cellulose membrane, an acetyl cellulose membrane, a polysulfone membrane, a polyethersulfone membrane, a nylon membrane, glass fiber, non-woven fabrics, a cloth, a thread or the like is preferable.

In the present invention, it is preferable that the labeling substance capturing area in which the second binding substance of the test substance or the binding substance of the first binding substance is immobilized be prepared on parts of the binding substance immobilized membrane. The second binding substance of the test substance or the binding substance of the first binding substance may be directly immobilized on parts of the binding substance immobilized membrane by physical or chemical binding, or may be physically or chemically bound to fine particles such as latex particles, these fine particles are trapped in parts of the binding substance immobilized membrane and immobilized, and the labeling substance capturing area is formed. In addition, it is preferable that, after the second binding substance or the binding substance of the first binding substance is immobilized, the binding substance immobilized membrane be treated for non-specific adsorption prevention by a treatment using an inactive protein and the like and used.

### 5-4. Labeling Substance Holding Pad

In the present invention, an embodiment in which a labeling substance holding pad having a labeling substance holding area is installed in the chromatographic kit and used is preferable. As the material of the labeling substance holding pad, for example, cellulose filter paper, glass fibers, non-woven cloth and the like may be preferably used, and a certain amount of the labeling substance prepared as described above is impregnated and dried, and this is made to be the labeling substance holding area.

### 5-5. Sample Addition Pad

In the chromatographic kit of the present invention, it is preferable that a sample addition pad be further installed and used. As the sample addition pad, an embodiment in which the sample addition pad not only accepts the sample including the test substance added but also has a function to filter insoluble particles and the like in the sample is preferable. The material of a sample addition pad may include a material having uniform properties such as cellulose filter paper, glass fiber, polyurethane, polyacetate, cellulose acetate, nylon and cotton cloth. In addition, in order to prevent the non-specific adsorption of the test substance in the sample to the material of the sample addition pad and lowering the degree of accuracy of the analysis at the time of the analysis, the material the sample addition unit is composed of is sometimes treated for non-specific adsorption prevention in advance and used. The sample addition pad may also serve as the labeling substance holding pad having a labeling substance holding area described in 5-4.

### 5-6. Water Absorption Pad

In the present invention, a water absorption pad may preferably be installed in the chromatographic kit and used. The water absorption pad is a site which absorbs and removes the unreacted labeling substance and the like which does not become insoluble in the detecting unit of the chromatographic carrier and at the same time, physically absorbs the added sample by chromatographic development, and an absorbent material such as cellulose filter paper, non-woven cloth, a cloth, cellulose acetate or the like is used. The speed of the chromatography after tip of the chromatographed added sample reaches the water absorption pad depends on the material, size or the like of the water absorption pad, therefore, the speed that matches the measurement of the test substance may be set by the selection.

### 6. Method of Immunity Test

Hereinafter, a sandwich-type method and a competition method as specific embodiments according to the chromatography method of the present invention is described.

In the sandwich-type method, while not particularly limited, for example, an analysis of the test substance may be carried out according to the following procedure. First, the first binding substance (for example, a first antibody) and the second binding substance having specificity with respect to a test substance (an antigen) are prepared in advance by the method described above. In addition, the labeling substance is modified in advance using the first binding substance. The second binding substance is immobilized on an appropriate binding substance immobilized membrane (for example, a nitrocellulose membrane, a glass fiber membrane, a nylon membrane, a cellulose membrane, or the like) and this is made to be a labeling substance capturing area, and the second binding substance is brought into contact with a test sample (or the extracted solution thereof) which has a possibility of including the test substance (an antigen), and binding with the second binding substance (an antigen-antibody reaction with a second antibody) occurs if the test substance is present in the test sample. When an excess of the labeling substance modified with the first binding substance is further brought into contact at the same time as or after the binding of the test substance and the second binding substance, a complex made of the immobilized second binding substance, the test substance (antigen) and the labeling substance modified with the first binding substance is formed if the test substance is present in the test sample.

In the sandwich-type method, after the reaction of the immobilized second binding substance with the test substance (an antigen) and the test substance with the first binding substance which modifies the labeling substance is complete, the labeling substance which did not form the immune complex is removed, and subsequently, for example, the labeling substance capturing area of the binding substance immobilized membrane is observed as is, the labeling substance is detected or quantified, the presence or absence or the amount of the test substance in the test sample may be measured. In the present invention, a signal from the labeling substance which forms such a complex is amplified and detected by supplying, for example, a reducing agent and a silver ion-containing compound.

In the competition method, while not particularly limited, for example, the test substance may be analyzed by the following procedure. A competition method is well known in the art as a method to detect antigens of low molecular weight compounds which may not be assayed using a sandwich method. First, the first binding substance having specificity with respect to the test substance (an antigen) is prepared in advance. In addition, the labeling substance is modified in advance using the first binding substance. The test substance itself which is the binding substance of the first binding substance or a similar compound of the test substance having a similar site with the test substance is immobilized on an appropriate binding substance immobilized membrane (for example, a nitrocellulose membrane, a glass fiber membrane, a nylon membrane, a cellulose membrane, or the like), and this is made to be a labeling substance capturing area. If a test sample (or the extracted solution thereof) which has a possibility of including the test substance (an antigen) is brought into contact with the labeling substance modified in advance with the first binding substance, a complex is formed by the first binding substance which modifies the labeling substance and the test substance itself or a similar compound of the test substance having a binding property with respect to the first binding substance immobilized on the labeling substance capturing area if the test substance is not present in the test sample. On the other hand, if the test substance is present in the test sample, the test substance (antigen) binds to the first binding substance which modifies the labeling substance, therefore, subsequent binding to the test substance itself or a similar compound of the test substance having a binding property with respect to the first binding substance which is immobilized on the labeling substance capturing area is inhibited, and a complex is not formed.

After the reaction between the test substance itself or a similar compound having a similar site with the test substance which is immobilized on the second binding substance and the first binding substance which modifies the labeling substance is complete, the first binding substance which modifies labeling substance which did not bind is removed, and subsequently, the amount of the labeling substance modified with the first binding substance in the labeling substance capturing area is detected or quantified, therefore, the presence or absence or the amount of the test substance in the test sample may be measured. In the present invention, a signal from the labeling substance which forms such a complex is amplified and detected by supplying, for example, a reducing agent and a silver ion-containing compound to the area immobilized.

### 7. Amplification Liquid

An amplification liquid is a solution in which chemicals included are catalytically reacted by an action of the labeling substance or the test substance and therefore generates a colored compound, luminescence or the like and may cause an amplification of the signal. For example, on a metallic label, a silver ion solution which causes a precipitation of the metallic silver by a physical photo-development or a solution of a phenylenediamine compound and a naphthol compound which becomes a dye due to an action of a peroxidase label and hydrogen peroxide may be included.

In detail, a so-called photo-developing solution described in general books in the field of photochemistry (for example, "Principles of Photographic Science and Engineering (revised), - Silver Salt Photography -" (The Society of Photography and Imaging of Japan, Corona Publishing Co., Ltd.), "Chemistry of Photography" (Akira Sasai, Photographic Industry Publication, Co., Ltd.) "Latest Prescription Handbook" (Shinichi Kikuchi et al., Amico Publishing Company) may be used, and a so-called physical photo-developing solution which includes silver ions in the solution and is reduced with the colloidal metal in which the silver ions in the solution becomes a core of the photo-development as a center may be used as the amplification liquid without being particularly limited.

In the present invention, two types of amplification reagents are used. It is preferable that, of the two types of the amplification reagents used to amplify the signal of the labeling substance captured in the labeling substance capturing area, the first amplification reagent be included in a first liquid and the second amplification reagent be included in a second liquid, and amplification be performed by sequentially adding the first liquid and the second liquid. The first liquid is preferably added to a pad to deliver the liquid of the reducing agent solution located further in an upstream direction than the labeling substance holding pad and the sample addition pad.

A combination of the first liquid which includes a reducing agent for silver ions and the second liquid which includes a compound including silver ions is used.

Hereinafter, the reducing agent for silver ions included in the first liquid and the compound which includes silver ions included in the second liquid, and the like, are described.

### 7-1 Compound Including Silver Ions

As the compound including silver ions, an organic silver salt, an inorganic silver salt or a silver complex may be used. The compound including silver ions with high-solubility in solvents such as water is preferable, and silver nitrate, silver acetate, silver lactate, silver butyrate, silver thiosulfate or the like may be included. Silver nitrate is particularly preferable. As the silver complex, a silver complex coordinated to a ligand having a water-soluble group such as a hydroxyl group or a sulfonic group is preferable, and silver hydroxy thioether or the like may be included.

It is preferable that the inorganic silver salt or the silver complex be included generally at 0.001 mol/m² to 0.2 mol/m² and preferably at 0.01 mol/m² to 0.05 mol/m² as silver.

### 7-2 Reducing Agent for Silver Ions

The reducing agent for silver ions used includes divalent iron ions. When using such a reducing agent, it is necessary that the iron ions oxidized are either removed or made to be harmless by complex formation or reduction. Fe²⁺ may be made to be harmless by forming a complex of Fe³⁺ which is an oxide, using citric acid or EDTA.

### 8. Other auxiliaries

As other auxiliaries of the amplification liquid, a buffering agent, a preservative, for example, an antioxidant or an organic stabilizer, or a speed adjusting agent may be included. As the buffering agent, for example, a buffering agent which uses acetic acid, citric acid, sodium hydroxide or a salt of any of these or tris(hydroxymethyl)aminomethane, or other buffering agents used in general chemical experiments may be used. These buffering agents are used appropriately and the pH may be adjusted to be optimized as the amplification liquid. Also, an alkylamine as an antifogging agent may be used as an additive and dodecylamine is particularly preferable. Also, in order to enhance the solubility of these additives, the surfactant may be used and C₉H₁₉-C₆H₄-O-(CH₂CH₂O)₅₀H is particularly preferable.

As a method in which the amplification reagent is spotted on the chromatographic kit, a method in which the reducing agent solution as the first liquid is spotted on the pad to deliver the liquid of the reducing agent solution, and the silver ion solution as the second liquid is spotted in the labeling substance capturing area from the top is preferable.

The method in which the two types of the amplification reagents are included inside the chromatographic kit includes a method in which a pot including a solution containing each amplification reagent is disposed at the top of the site in which each amplification reagent is spotted. It is preferable that a pot including the silver ion solution (a second liquid) be provided on the top of a silver ion solution filling hole by placing the reducing agent solution (a first liquid) at the top of the pad to deliver the liquid of the reducing agent solution. From these arrangements, the liquid may flow by pressing each pot and may be spotted at predetermined sites.

The present invention is described more specifically by the following examples, however, the present invention is not limited to these examples.

### [Examples]

### (1) Preparation of Immunochromatographic Kit for Influenza Virus Antigen Detection

### (1-1) Preparation of Colloidal Gold Modified with Anti-influenza Type A Monoclonal Antibody (Labeling Substance Modified with First Binding Substance of Test Substance)

1 mL of 160 µg/mL anti-influenza type A monoclonal antibody (Anti-Influenza A SPTN-5 7307, Medix Biochemica) solution was added to the colloidal gold solution of which pH was adjusted by adding 1 mL of 50 mM KH₂PO₄ buffer (pH 7.5) to 9 mL of colloidal gold solution with a diameter of 50 nm (EM. GC50, BBI Corporation) and the mixture was stirred. After standing for 10 minutes, 550 µL of 1 % by mass aqueous solution of polyethylene glycol (PEG Mw. 20,000, Product No. 168-11285, manufactured by Wako Pure Chemical Industries, Ltd.) was added and the mixture was stirred, and subsequently 1.1 mL of 10 % by mass aqueous solution of bovine serum albumin (BSA FractionV, Product No. A-7906, manufactured by SIGMA) was added and the mixture was stirred. After this solution was centrifuged (himacCF16RX, manufactured by Hitachi, Ltd.) for 30 minutes under the condition of 8,000 xg and 4°C, supernatant was removed leaving approximately 1 mL and the colloidal gold was re-dispersed using an ultrasonic cleaner. After that, the resultant was dispersed to 20 mL of colloidal gold preservative solution (20 mM Tris-HCl buffer (pH 8.2), 0.05 % by mass PEG (Mw. 20,000), 150 mM NaCl, 1 % by mass BSA), was centrifuged again for 30 minutes under the condition of 8,000 xg and 4°C, supernatant was removed leaving approximately 1 mL and the colloidal gold was re-dispersed using an ultrasonic cleaner, and the solution of the colloidal gold modified with antibody (50 nm) was obtained.

### (1-2) Preparation of Colloidal Gold Holding Pad (Labeling Substance Holding Area)

Colloidal gold modified with anti-influenza type A monoclonal antibody made in (1-1) was diluted by colloidal gold coating liquid (20 mM Tris-HCl buffer (pH 8.2), 0.05 % by mass PEG (Mw. 20,000), 5 % by mass sucrose) and water, and was diluted so that the optical density (OD) at 520 nm became 0.1. This solution was uniformly coated on a glass fiber pad (Glass Fiber Conjugate Pad, Millipore Corporation) which was cut into 8 mmx150 mm with 0.8 mL per each pad, and after dried under reduced pressure for 12 hours and cut into 5 mm, the colloidal gold holding pad modified with anti-influenza type A monoclonal antibody was obtained. The portion which holds the colloidal gold antibody corresponds to the labeling substance holding area.

### (1-3-1) Preparation of Antibody Immobilized Membrane-1 (Binding Substance Immobilized Membrane-1)

An antibody immobilized membrane-1 was made by immobilizing the antibody according to the following method with respect to the nitrocellulose membrane which was cut into 60 mmx300 mm (using a plastic backing, HiFlow Plus HF120, manufactured by Millipore Corporation). The long side of the membrane was set as down, and at the position of 15 mm from the bottom, the solution of anti-influenza type A monoclonal antibody (Anti-Influenza A SPTN-5 7307, manufactured by Medix Biochemica) prepared to be 1.5 mg/mL was coated in a line shape and was made to be the antibody immobilized line. In addition, at the position of 11 mm from the bottom, the solution of anti-mouse IgG antibody (anti-mouse IgG (H+L), rabbit F(ab')2, Product No. 566-70621, manufactured by Wako Pure Chemical Industries, Ltd.) prepared to be 0.2 mg/mL was coated in a line shape. The coated membrane was dried for 30 minutes at 50°C using a warm air dryer. 500 mL of blocking liquid (50 mM boric acid buffer (pH 8.5) containing 0.5 % by mass casein (derived from milk, Product No. 030-01505, manufactured by Wako Pure Chemical Industries, Ltd.)) was placed in a vat and was allowed to stand for 30 minutes as is. After that, the membrane was transferred and immersed in 500 mL of cleaning and stabilizing liquid (50 mM Tris-HCl (pH 7.5) buffer including 0.5 % by mass sucrose and 0.05 % by mass sodium cholate) placed in a similar vat and was allowed to stand for 30 minutes as is. The membrane was taken out from the liquid, dried for 12 hours at room temperature, was cut into a width of 5 mm and this was made to be the antibody immobilized membrane 1. The portion in which the anti-influenza type A monoclonal antibody is immobilized corresponds to the labeling substance capturing area, and the portion in which the anti-mouse IgG antibody is immobilized corresponds to a positive control area. In addition, an assay using the sandwich method is possible even when the labeled antibody and the immobilized antibody are the same since the protein recognized by the anti-influenza type A monoclonal antibody used in the present example is a trimer of the same unit.

### (1-3-2) Preparation of Antibody Immobilized Membrane-2 (Binding Substance Immobilized Membrane-2)

An antibody immobilized membrane-2 was made by immobilizing the antibody and the color developing reagent according to the following method with respect to the nitrocellulose membrane which was cut into 60 mmx300 mm (using a plastic backing, HiFlow Plus HF120, manufactured by Millipore Corporation). The long side of the membrane was set as down, and at the position of 15 mm from the bottom, the solution of anti-influenza type A monoclonal antibody (Anti-Influenza A SPTN-5 7307, manufactured by Medix Biochemica) prepared to be 1.5 mg/mL was coated in a line shape and was made to be the antibody immobilized line. In addition, at the position of 11 mm from the bottom, the solution of anti-mouse IgG antibody (anti-mouse IgG (H+L), rabbit F(ab')2, Product No. 566-70621, manufactured by Wako Pure Chemical Industries, Ltd.) prepared to be 0.2 mg/mL was coated in a line shape. The coated membrane was dried for 30 minutes at 50°C using a warm air dryer. 500 mL of blocking liquid (50 mM boric acid buffer (pH 8.5) containing 0.5 % by mass casein (derived from milk, Product No. 030-01505, manufactured by Wako Pure Chemical Industries, Ltd.)) was placed in a vat and was allowed to stand for 30 minutes as is. After that, the membrane was transferred and immersed in 500 mL of cleaning and stabilizing liquid (50 mM Tris-HCl (pH 7.5) buffer including 0.5 % by mass sucrose and 0.05 % by mass sodium cholate) placed in a similar vat and was allowed to stand for 30 minutes as is. The membrane was taken out from the liquid, dried for 12 hours at room temperature. At the position of 9 mm from the bottom, bathophenanthroline (manufactured by Dojindo Laboratories) adjusted to be 20 mM was coated in a line shape and was made to be the color developing reagent immobilized line. The membrane was cut into a width of 5 mm and this was made to be the antibody immobilized membrane-2. The portion in which the anti-influenza type A monoclonal antibody is immobilized corresponds to the labeling substance capturing area, and the portion in which the anti-mouse IgG antibody is immobilized corresponds to a positive control area.

### (1-3-3) Preparation of Antibody Immobilized Membrane-3 (Binding Substance Immobilized Membrane-3)

An antibody immobilized membrane-3 was made by immobilizing the antibody and the color developing reagent according to the following method with respect to the nitrocellulose membrane which was cut into 60 mmx300 mm (using a plastic backing, HiFlow Plus HF120, manufactured by Millipore Corporation). The long side of the membrane was set as down, and at the position of 15 mm from the bottom, the solution of anti-influenza type A monoclonal antibody (Anti-Influenza A SPTN-5 7307, manufactured by Medix Biochemica) prepared to be 1.5 mg/mL was coated in a line shape and was made to be the antibody immobilized line. In addition, at the position of 11 mm further from the bottom, the solution of anti-mouse IgG antibody (anti-mouse IgG (H+L), rabbit F(ab')2, Product No. 566-70621, manufactured by Wako Pure Chemical Industries, Ltd.) prepared to be 0.2 mg/mL was coated in a line shape. At the position of 9 mm from the bottom, bromocresol green (manufactured by Wako Pure Chemical Industries, Ltd.) adjusted to be 30 mM was coated in a line shape and was made to be the color developing reagent immobilized line. The coated membrane was dried for 30 minutes at 50°C using a warm air dryer. 500 mL of blocking liquid (50 mM boric acid buffer (pH 8.5) containing 0.5 % by mass casein (derived from milk, Product No. 030-01505, manufactured by Wako Pure Chemical Industries, Ltd.)) was placed in a vat and was allowed to stand for 30 minutes as is. After that, the membrane was transferred and immersed in 500 mL of cleaning and stabilizing liquid (50 mM Tris-HCl (pH 7.5) buffer including 0.5 % by mass sucrose and 0.05 % by mass sodium cholate) placed in a similar vat and was allowed to stand for 30 minutes as is. The membrane was taken out from the liquid, dried for 12 hours at room temperature, and was cut into a width of 5 mm and this was made to be the antibody immobilized membrane-3. The portion in which the anti-influenza type A monoclonal antibody is immobilized corresponds to the labeling substance capturing area, and the portion in which the anti-mouse IgG antibody is immobilized corresponds to a positive control area.

### (1-4) Preparation of Silver Amplification Liquid

### (1-4-1) Preparation of Reducing Agent Solution (First Amplification Reagent)

23.6 ml of 1 mol/l aqueous solution of iron nitrate made by dissolving iron(III) nitrate nonahydrate (manufactured by Wako Pure Chemical Industries, Ltd., 095-00995) in water and 13.1 g of citric acid (manufactured by Wako Pure Chemical Industries, Ltd., 038-06925) were dissolved in 290 g of water. When these were all dissolved, 36 ml of nitric acid (10 % by mass) was added stirring using a stirrer, 60.8 g of iron(II) ammonium sulfate hexahydrate (manufactured by Wako Pure Chemical Industries, Ltd., 091-00855) was added thereto, and this was made to be the reducing agent solution.

### (1-4-2) Preparation of Silver Ion Solution (Second Amplification Reagent)

8 ml of silver nitrate solution (including 10 g of silver nitrate) and 24 ml of 1 mol/l aqueous solution of iron nitrate were added to 66 g of water. Furthermore, this solution and a solution in which 5.9 ml of nitric acid (10 % by mass), 0.1 g of dodecylamine (manufactured by Wako Pure Chemical Industries, Ltd., 123-00246), and 0.1 g of surfactant C₁₂H₂₅-C₆H₄-O-(CH₂CH₂O)₅₀H were dissolved in advance in 47.6 g of water were mixed, and this was made to be the silver ion solution.

### (1-5) Preparation of 1,10-Phenanthroline-Containing Water Absorption Pad (Area Having Color Developing Reagent)

A water absorption pad (GB-140, ADVANTEC MFS, Inc.) was cut into 150 mmX100 mm and was immersed in a 1,10-phenanthroline solution (21 mM). The water absorption pad was taken out from the 1,10-phenanthroline solution and dried under reduced pressure for 12 hours, and the phenanthroline-containing water absorption pad was obtained. The area containing phenanthroline corresponds to an area having the color developing reagent.

### (1-6-1) Preparation of Kit-1 for Assay

A schematic diagram of the kit components for assay is shown in Fig. 1. The material of the upper portion and lower portion constituting the outside of the kit was prepared by injection molding using polypropylene. The antibody immobilized membrane-1 (the anti-influenza type A antibody and the anti-mouse IgG antibody immobilized membrane) prepared in (1-3-1) as shown in Fig. 1, the water absorption pad which was not immersed in the color developing reagent (GB-140, manufactured by manufactured by ADVANTEC MFS, Inc., cut into 100 mmX 150 mm), the pad to deliver the liquid of the reducing agent solution (Glass Fiber Conjugate Pad, manufactured by Millipore Corporation), and the colloidal gold holding pad modified with the anti-influenza type A monoclonal antibody prepared in (1-2) at the center as the colloidal gold holding pad were loaded as shown in Fig. 1 and the kit-1 for assay (Comparative Example-1) was prepared.

### (1-6-2) Preparation of Kit-2 for Assay

The kit-2 for assay (Example-1) was prepared in the same manner as the preparation of the kit-1 for assay in (1-6-1) except that the water absorption pad containing 1,10-phenanthroline prepared in (1-5) was loaded instead of the water absorption pad which was not immersed in the color developing reagent.

### (1-6-3) Preparation of Kit-3 for Assay

A schematic diagram of the kit components for assay is shown in Fig. 2. The material of the upper portion and lower portion constituting the outside of the kit was prepared by injection molding using polypropylene. The antibody immobilized membrane-2 (the color developing reagent, the anti-influenza type A monoclonal antibody and the anti-mouse IgG antibody immobilized membrane) prepared in (1-3-2) as shown in Fig. 2, the water absorption pad which did not contain the color developing reagent (GB-140, manufactured by ADVANTEC MFS, Inc., cut into 100 mmX 150 mm), the pad to deliver the liquid of the reducing agent solution (Glass Fiber Conjugate Pad, manufactured by Millipore Corporation), and the colloidal gold holding pad modified with the anti-influenza type A monoclonal antibody prepared in (1-2) at the center as the colloidal gold holding pad were disposed and the kit-3 for assay (Example-2) was prepared.

### (1-6-4) Preparation of Kit-4 for Assay

The kit-4 for assay (Example-3) was prepared in the same manner as the preparation of the kit-3 for assay in (1-6-3) except that the antibody immobilized membrane-3 (the color developing reagent, the anti-influenza type A monoclonal antibody and the anti-mouse IgG antibody immobilized membrane) prepared in (1-3-3) was loaded instead of the antibody immobilized membrane-2 prepared in (1-3-2).

### (2) Evaluation

### (2-1) Development of Test Sample Liquid

The test sample liquid was prepared by diluting a simulated positive analyte (BD Flu examen control A+B- (manufactured by Becton, Dickinson and Company)) 128 times using an extract (1% by mass BSA-PBS 1 containing 1% by mass BIGCHAP). 30 µL of this test sample liquid was spotted to the colloidal gold holding pad modified with the anti-influenza type A monoclonal antibody of kit-1 to -4 for assay prepared in (1-6-1) to (1-6-4), and each test sample liquid was developed.

### (2-2) Development of Reducing Agent Solution

In (2-1), immediately after spotting the test sample liquid, the reducing agent solution was developed by spotting 200 µL of the reducing agent solution prepared in (1-4-1) on the pad to deliver the liquid of the reducing agent solution of kit-1 to -4 for assay.

### (3) Silver Amplification

### (3-1) Silver Amplification Initiation Signal is Absent (Comparative Example 1)

After spotting the reducing agent solution using the method described in (2-2), the silver amplification was carried out by adding 95 µL of the silver ion solution prepared in (1-4-2) from the silver ion solution filling hole of the device-1 to -4 for assay at an arbitrary timing, and blackening of the labeling substance replenishing area was observed four minutes after the spotting. This experiment was performed respectively by 10 different people, an amplified black line being acknowledged was considered to be a success, and the success rate was calculated. The results are shown in Table 2.

### (3-2) Silver Amplification Initiation Signal is Present (Example 1)

After spotting the reducing agent solution using the method described in (2-2) on the kit-2 for assay in which the water absorption pad containing 1,10-phenanthroline was used, the amplification was carried out by adding silver ion solution in the same manner as in (3-1) immediately after the red signal was observed in the water absorption pad containing 1,10-phenanthroline. This experiment was performed respectively by 10 different people, an amplified black line being acknowledged was considered to be a success, and the success rate was calculated. The results are shown in Table 2.

### (3-3) Silver Amplification Initiation Signal is Present (Example 2)

After spotting the reducing agent solution in the same manner as in (2-2) on the kit-3 for assay immobilizing bathophenanthroline which is the color developing reagent prepared in (1-6-3), the amplification was carried out by adding silver ion solution in the same manner as in (3-1) immediately after the line coated with bathophenanthroline which is the color developing reagent was developed in red color. This experiment was performed respectively by a plurality of different people, an amplified black line being acknowledged was considered to be a success, and the success rate was calculated. The results are shown in Table 2.

### (3-4) Silver Amplification Initiation Signal is Present (Example 3)

After spotting the reducing agent solution in the same manner as in (2-2) on the kit-4 for assay immobilizing bromocresol green which is the color developing reagent prepared in (1-6-4), the amplification was carried out by adding silver ion solution in the same manner as in (3-1) immediately after the color of the line coated with bromocresol green which is the color developing reagent was changed from deep green to orange. This experiment was performed respectively by a plurality of different people, an amplified black line being acknowledged was considered to be a success, and the success rate was calculated. The results are shown in Table 2.

**[Table 2]**

| | Silver Amplification Initiation Signal is Absent (Comparative Example 1) | Silver Amplification Initiation Signal is Present (Example 1) | Silver Amplification Initiation Signal is Present (Example 2) | Silver Amplification Initiation Signal is Present (Example 3) |
|---|---|---|---|---|
| Membrane | 1 | 1 | 2 | 3 |
| Kit for Assay | 1 | 2 | 3 | 4 |
| Color Developing Reagent | None | 1,10-phenanthro line | bathophenanthro line | bromocresol green |
| Amplification Success Rate | 20% | 100% | 100% | 100% |

From the results in Table 2, eliminating the failure of amplification was succeeded by enabling a visually determination of the timing at which amplification is carried out. The effects of the present invention were evident from these results.

## Claims

1. A chromatographic kit for detecting a test substance in a sample, the kit comprising:
(1) a labelling substance holding area, which is an area having a labelling substance bound to a first binding substance, wherein the labelling substance is a colloidal metal and the first binding substance is a substance capable of binding to the test substance;
(2) a labelling substance capturing area, which is an area having a second binding substance, wherein the second binding substance is a substance capable of binding to the test substance or to the first binding substance;
(3) first and second amplification reagents, which are capable of amplifying a signal of the labelling substance in order to aid the detection of the labelling substance; and
(4) an area having a colour developing reagent, which is a reagent capable of detecting the first amplification reagent;
wherein the first amplification reagent is a reducing agent for silver ions that includes divalent iron ions, the second amplification reagent is a compound including silver, and the colour developing agent is a compound having a phenanthroline skeleton, a compound having a bipyridine skeleton or a salton-based colour developing reagent.

2. A chromatographic kit according to Claim 1, wherein the colour developing reagent is a compound having a phenanthroline skeleton.

3. A chromatographic kit according to Claim 1, wherein the colloidal metal is colloidal gold.

4. A chromatographic kit according to any preceding claim, wherein:
the labelling substance holding area is a pad holding the labelling substance;
the labelling substance capturing area is located in a membrane in which the second binding substance is immobilized;
the colour developing reagent is carried on the membrane in which the second binding substance is immobilized; and
the kit further comprises a water absorption pad.

5. The chromatographic kit according to Claim 4, wherein the colour developing reagent is a colour developing reagent which does not substantially move in the binding substance immobilized membrane when any of an aqueous solution including the test sample or an aqueous solution including the first amplification reagent is developed.

6. A chromatography method using a chromatographic kit according to Claim 1, the method comprising:
(1) forming a complex between the test substance and the labelling substance bound to the first binding substance, and then capturing the labelling substance in the labelling substance capturing area;
(2) filling the labelling substance capturing area with the first amplification reagent from an upstream side of the labelling substance capturing area to a downstream side of the labelling substance capturing area;
(3) confirming that the labelling substance capturing area is filled with the first amplification reagent by detecting physical or chemical changes in the area having the colour developing reagent; and
(4) filling the labelling substance capturing area with the second amplification reagent after confirming that the labelling substance capturing area is filled with the first amplification reagent;
wherein the first amplification reagent is a reducing agent for silver ions that includes divalent iron ions, the second amplification reagent is a compound including silver, and the colour developing agent is a compound having a phenanthroline skeleton, a compound having a bipyridine skeleton or a salton-based colour developing reagent.

## Patentansprüche

1. Chromatographiekit zum Detektieren einer Testsubstanz in einer Probe, wobei das Kit umfasst:
(1) einen Bereich, der eine Markierungssubstanz aufnimmt, der ein Bereich ist, der eine Markierungssubstanz aufweist, die an eine erste bindende Substanz gebunden ist, worin die Markierungssubstanz ein kolloidales Metall ist und die erste bindende Substanz eine Substanz ist, die in der Lage ist, an die Testsubstanz zu binden;
(2) einen Bereich, der eine Markierungssubstanz einfängt, der ein Bereich ist, der eine zweite bindende Substanz aufweist, worin die zweite bindende Substanz eine Substanz ist, die in der Lage ist, an die Testsubstanz oder an die erste bindende Substanz zu binden;
(3) erste und zweite Verstärkungsreagentien, die in der Lage sind, ein Signal der Markierungssubstanz zu verstärken, um die Detektion der Markierungssubstanz zu unterstützen; und
(4) einen Bereich, der ein Farbentwicklungsreagens aufweist, das ein Reagenz ist, das in der Lage ist, das erste Verstärkungsreagens zu detektieren;
worin das erste Verstärkungsreagens ein Reduktionsmittelfür Silberionen ist, das zweiwertige Eisenionen einschließt, das zweite Verstärkungsreagens eine Verbindung ist, die Silber einschließt, und das Farbentwicklungsmittel eine Verbindung, die ein Phenanthrolingerüst aufweist, eine Verbindung, die ein Bipyridingerüst aufweist, oder ein Salton-basiertes Farbentwicklungsreagens ist.

2. Chromatographiekit gemäß Anspruch 1, worin das Farbentwicklungsreagens eine Verbindung ist, die ein Phenanthrolingerüst aufweist.

3. Chromatographiekit gemäß Anspruch 1, worin das kolloidale Metall kolloidales Gold ist.

4. Chromatographiekit gemäß einem beliebigen vorherigen Anspruch, worin:
der Bereich, der die Markierungssubstanz aufnimmt, ein Kissen ist, das die Markierungssubstanz aufnimmt;
der Bereich, der die Markierungssubstanz einfängt, sich in einer Membran befindet, in der die zweite bindende Substanz immobilisiert ist;
das Farbentwicklungsreagens auf der Membran geträgert ist, in der die zweite bindende Substanz immobilisiert ist; und
das Kit ferner ein Kissen für die Wasserabsorption umfasst.

5. Chromatographiekit gemäß Anspruch 4, worin das Farbentwicklungsreagens ein Farbentwicklungsreagens ist, das sich im wesentlichen nicht in der Membran mit der immobilisierten bindenden Substanz bewegt, wenn eine beliebige aus einer wässrigen Lösung, die die Testprobe einschließt, oder einer wässrigen Lösung, die das erste Verstärkungsreagens einschließt, entwickelt wird.

6. Chromatographieverfahren, das ein Chromatographiekit gemäß Anspruch 1 verwendet, wobei das Verfahren umfasst:
(1) Bilden eines Komplexes zwischen der Testsubstanz und der Markierungssubstanz, die an die erste bindende Substanz gebunden ist, und dann Einfangen der Markierungssubstanz in dem Bereich, der die Markierungssubstanz einfängt;
(2) Befüllen des Bereichs, der die Markierungssubstanz einfängt, mit dem ersten Verstärkungsreagens von einer stromaufwärts gelegenen Seite des Bereichs, der die Markierungssubstanz einfängt, zu einer stromabwärts gelegenen Seite des Bereichs, der die Markierungssubstanz einfängt;
(3) Bestätigen, dass der Bereich, der die Markierungssubstanz einfängt, mit dem ersten Verstärkungsreagens gefüllt ist durch Detektion physikalischer oder chemischer Veränderungen in dem Bereich, der das Farbentwicklungsreagens aufweist; und
(4) Befüllen des Bereichs, der die Markierungssubstanz einfängt, mit dem zweiten Verstärkungsreagens, nach Bestätigen, dass der Bereich, der die Markierungssubstanz einfängt, mit dem ersten Verstärkungsreagens befüllt ist;
worin das erste Verstärkungsreagens ein Reduktionsmittel für Silberionen ist, das zweiwertige Eisenionen einschließt, das zweite Verstärkungsreagens eine Verbindung ist, die Silber einschließt, und das Farbentwicklungsmittel eine Verbindung ist, die ein Phenanthrolingerüst aufweist, eine Verbindung, die ein Bipyridingerüst aufweist, oder ein Salton-basiertes Farbentwicklungsreagens ist.

## Revendications

1. Kit chromatographique destiné à détecter une substance d'essai dans un échantillon, le kit comprenant :
(1) une zone de maintien de substance de marquage, qui est une zone ayant une substance de marquage liée à une première substance de liaison, où la substance de marquage est un métal colloïdal et la première substance de liaison est une substance capable de se lier à la substance d'essai ;
(2) une zone de capture de substance de marquage, qui est une zone ayant une deuxième substance de liaison, où la deuxième substance de liaison est une substance capable de se lier à la substance d'essai ou à la première substance de liaison ;
(3) des premier et deuxième réactifs d'amplification, qui sont capables d'amplifier un signal de la substance de marquage afin de faciliter la détection de la substance de marquage ; et
(4) une zone ayant un réactif de développement de couleur, qui est un réactif capable de détecter le premier réactif d'amplification ;
dans lequel le premier réactif d'amplification est un agent réducteur pour des ions argent qui comporte des ions fer divalents, le deuxième réactif d'amplification est un composé comportant de l'argent, et l'agent de développement de couleur est un composé ayant un squelette de phénanthroline, un composé ayant un squelette de bipyridine ou un réactif de développement de couleur blanc argenté.

2. Kit chromatographique selon la revendication 1, dans lequel le réactif de développement de couleur est un composé ayant un squelette de phénanthroline.

3. Kit chromatographique selon la revendication 1, dans lequel le métal colloïdal est de l'or colloïdal.

4. Kit chromatographique selon l'une des revendications précédentes, dans lequel :
la zone de maintien de substance de marquage est un tampon maintenant la substance de marquage ;
la zone de capture de substance de marquage est située dans une membrane dans laquelle la deuxième substance de liaison est immobilisée ;
le réactif de développement de couleur est porté sur la membrane dans laquelle la deuxième substance liaison est immobilisée ; et
le kit comprend en outre un tampon d'absorption d'eau.

5. Kit chromatographique selon la revendication 4, dans lequel le réactif de développement de couleur est un réactif de développement de couleur qui ne se déplace pas substantiellement dans la membrane à substance de liaison immobilisée lorsqu'une solution quelconque d'une solution aqueuse comportant l'échantillon d'essai et d'une solution aqueuse comportant le premier réactif d'amplification est développée.

6. Procédé de chromatographie utilisant un kit chromatographique selon la revendication 1, le procédé comprenant le fait :
(1) de former un complexe entre la substance d'essai et la substance de marquage liée à la première substance de liaison, et de capturer ensuite la substance de marquage dans la zone de capture de substance de marquage ;
(2) de remplir la zone de capture de substance de marquage avec le premier réactif d'amplification depuis un côté amont de la zone de capture de substance de marquage à un côté aval de la zone de capture de substance de marquage ;
(3) de confirmer que la zone de capture de substance de marquage est remplie avec le premier réactif d'amplification en détectant des changements physiques ou chimiques dans la zone ayant le réactif de développement de couleur ; et
(4) de remplir la zone de capture de substance de marquage avec le deuxième réactif d'amplification après avoir confirmé que la zone de capture de substance de marquage est remplie avec le premier réactif d'amplification ;
dans lequel le premier réactif d'amplification est un agent réducteur pour des ions argent qui comporte des ions fer divalents, le deuxième réactif d'amplification est un composé comportant de l'argent, et l'agent de développement de couleur est un composé ayant un squelette de phénanthroline, un composé ayant un squelette de bipyridine ou réactif de développement de couleur blanc argenté.
